# EUROPEAN PATENT APPLICATION

(11) **EP 4 813 375 A1**
(43) Date of publication of application: **30.09.2026**
(21) Application number: 25216844.8
(22) Date of filing: 19.11.2025
(51) Int. Cl.: A61K 9/20, A61K 9/28, A61K 31/496

(54) **A FILM COATED TABLET FORMULATION COMPRISING AVATROMBOPAG**

(30) Priority: 24.03.2025 TR 2025003510
(71) Applicant: Sanovel Ilac Sanayi Ve Ticaret Anonim Sirketi, Istanbul (TR)
(72) Inventor: SUNEL, Fatih, Istanbul (TR); ATAK, Fadime Bilgehan, Istanbul (TR); GULER, Tolga, Istanbul (TR); SUEKINCI, Sinan, Istanbul (TR)
(74) Representative: Genç Ilhan, Oznur

(57) **Abstract**

The present invention relates to a film coated tablet formulation comprising avatrombopag maleate or a pharmaceutically acceptable salt, amorphous or crystalline form thereof and at least one filler wherein avatrombopag maleate is crystalline form B. The present invention also relates to a simple, rapid, cost effective, time-saving and industrially convenient method of preparing the formulation.

## Description

### Field of the Invention

The present invention relates to a film coated tablet formulation comprising avatrombopag maleate or a pharmaceutically acceptable salt, amorphous or crystalline form thereof and at least one filler wherein avatrombopag maleate is crystalline form B. The present invention also relates to a simple, rapid, cost effective, time-saving and industrially convenient method of preparing the formulation.

### Background of the Invention

Avatrombopag, sold under the brand name Doptelet, is a medication that used for certain conditions that lead to thrombocytopenia (low platelets) such as thrombocytopenia associated with chronic liver disease in adults who are to undergo a planned medical or dental procedure. The molecular formula of avatrombopag maleate is C₂₉H₃₄Cl₂N₆O₃S₂·C₄H₄O₄, and its structural formula(I) is as follows:

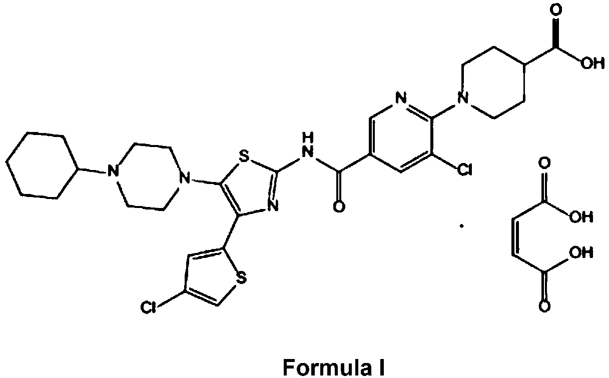

Avatrombopag is a white to off white powder with a nonchiral structure. The compound is practically insoluble in water and is nonhygroscopic. However, avatrombopag maleate is almost insoluble in water and 0.1M hydrochloric acid and is almost insoluble in the pH range of all systems in the body.

Avatrombopag maleate is an oral, small molecule thrombopoietin (TPO) receptor (c-Mpl) agonist, which can simulate the biological effect of TPO, stimulate platelet production, improve platelet count, belonging to the clinical urgent need medicine.

The patent CN112022825A discloses avatrombopag maleate tablet and its preparation method. In this patent, the crystalline form of Avatrombopag maleate is Form C.

The patent CN118453530A discloses an avatrombopag maleate tablet and a preparation method of the avatrombopag maleate tablet. The acridol maleate tablet comprises a tablet core and a film coating layer, the tablet core part of the medicine comprises acridol maleate (the particle size D90 is less than or equal to 85.6 mu m), a filler is anhydrous lactose and lactose in a ratio of (4.6: 1)-(3: 1), a disintegrating agent and a lubricating agent adopt two modes of internal and external addition, the internal and external addition ratio of the disintegrating agent is (2: 1)-(4: 1), and the internal and external addition ratio of the lubricating agent is (1: 1)-(1: 2.4). In this patent, the crystalline form of Avatrombopag maleate is Form C.

Avatrombopag generally used in solid form in the formulation, drug polymorphism is one of the important factors influencing the quality of medicine and clinical curative effect, therefore, has very important meaning to research of the crystal form.

There are many patent applications for avatrombopag in the prior art. There is still a need for a new and simple formulation and process. To overcome the low solubility of the active substance, the crystalline B form of avatrombopag maleate was used. When using the B form of avatrombopag maleate, a formulation with high solubility and thus the desired dissolution profile was obtained.

### Detailed description of the Invention

The main object of the present invention is to provide a film coated tablet formulation comprising avatrombopag maleate desired dissolution profile.

Another object of the present invention is to provide a film coated tablet formulation comprising avatrombopag maleate which has the content uniformity and flowability.

Another object of the present invention is to provide a preparation process of a film coated tablet formulation comprising avatrombopag maleate which is simple and cost-effective process.

Avatrombopag maleate exhibits low solubility in water, which can make it difficult to formulate in oral solid dosage forms like tablets or capsules without the use of solubility-enhancing techniques. Because avatrombopag maleate is poorly soluble in water, its dissolution rate can be slow. Therefore, there is still a need to develop a new formulation with high solubility.

In our studies, we tried various crystal forms of avatrombopag maleate, namely A, B and C. However, when we used form B of avatrombopag maleate, we saw that the solubility gave better results than forms A and C. Therefore, we obtained a formulation with high solubility by using form B of avatrombopag maleate. So, the formulation has the desired dissolution profile.

According to one embodiment of the invention, a film coated tablet formulation comprising avatrombopag maleate or a pharmaceutically acceptable salt, amorphous or crystalline form thereof and at least one filler wherein avatrombopag maleate is crystalline form B.

According to one embodiment of the invention, a film coated tablet formulation comprising avatrombopag maleate or a pharmaceutically acceptable salt, amorphous or crystalline form thereof and at least one disintegrant wherein avatrombopag maleate is crystalline form B.

According to this embodiment of the invention, the amount of avatrombopag maleate is between 1.0% and 20.0% by weight of the total formulation. Preferably, the amount of avatrombopag maleate is between 5.0% and 15.0% by weight of total formulation.

Suitable fillers are selected from the group comprising microcrystalline cellulose, lactose monohydrate, ammonium alginate, calcium carbonate, calcium phosphate, calcium phosphate dehydrate, neutral pellets, calcium sulfate, cellulose, cellulose acetate, compressible sugar, dextrates, dextrin, dextrose, ethylcellulose, fructose, glyceryl palmitostearate, lactitol, mannitol, polymethacrylates, simethicone, sodium alginate, sodium chloride, sorbitol, sucrose, sugar spheres, sulfobutylether beta-cyclodextrin, tragacanth, trehalose, xylitol or mixtures thereof.

According to one embodiment of the present invention, the filler is microcrystalline cellulose or lactose monohydrate or mixture thereof.

According to one embodiment of the present invention, the amount of filler is between 70.0% and 90.0% by weight of the total formulation. Preferably, the amount of filler is between 75.0% and 85.0% by weight of total formulation. This filler ratio ensures that the powder mixture flows smoothly during the compression process, resulting in a uniform tablet weight. Thus, eliminates problems such as flowability and content uniformity.

According to an embodiment of the present invention, the film coated tablet formulation further comprises at least one pharmaceutically acceptable excipient which is selected from the group comprising disintegrants, lubricants/glidants, film coating agents or a mixture thereof.

Suitable disintegrants are selected from the group comprising crospovidone, croscarmellose sodium, sodium starch glycolate, low-substituted hydroxypropyl cellulose, sodium carboxymethyl cellulose, calcium carboxymethyl cellulose, carboxymethyl cellulose, docusate sodium, guar gum, polyacrylate potassium, sodium alginate, corn starch, alginic acid, alginates, ion-exchange resins, magnesium aluminum silica, sodium dodecyl sulphate, poloxamer, sodium glycine carbonate, sodium lauryl sulphate or mixtures thereof.

According to one embodiment of the present invention, the disintegrant is crospovidone.

According to one embodiment of the present invention, the amount of disintegrant is between 0.1% and 10.0% by weight of the total formulation.

Suitable lubricants/glidants are selected from the group comprising from magnesium stearate, colloidal silicon dioxide, talc, calcium stearate, zinc stearate, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, sodium stearyl fumarate, sodium lauryl sulphate or mixtures thereof.

According to one embodiment of the present invention, the lubricant/glidant is magnesium stearate or colloidal silicon dioxide or mixture thereof.

According to one embodiment of the present invention, the amount of lubricant/glidant is between 0.01% and 10.0% by weight of the total formulation.

Suitable film coating agents are selected from the group comprising opadry yellow, polyvinyl alcohol (PVA), titanium dioxide, macrogol (PEG 3350), iron oxide, talc, Indigo carmine aluminium lake, carnauba wax, hydroxypropylmethylcellulose, polyethylene glycol (PEG), polyvinyl alcohol-polyethylene glycol copolymers, lecithin, polyvinylprolidone-vinyl acetate copolymer (PVP-VA), pigments, dyes or a mixture thereof.

According to this embodiment of the present invention, the amount of film coating agent is between 1.0% and 8.0% by weight of the total formulation.

According to one embodiment of the present invention, a film coated tablet formulation comprising avatrombopag maleate or a pharmaceutically acceptable salt, amorphous and crystalline form thereof is obtained by direct compression process. In direct compression, a film coated tablet ingredients are not exposed to moisture, solvents and heat. Thus, in this invention, direct compression is used to process moisture, solvent and/or heat sensitive avatrombopag.

A process for preparing a film coated tablet formulation comprising avatrombopag comprises the following steps;
a) Mixing avatrombopag Maleate form B, fillers, disintegrant and lubricant/glidant,
b) Sieving the mixture in step (a),
c) Adding lubricant/glidant to the mixture in step (b),
d) Compressing the homogeneous mixture obtained in such a way that each tablet is of the specified specifications,
e) Coating the core tablet with film coating.

### Example 1;

| **Ingredients** | **%by weight** | **%by weight** |
|---|---|---|
| Avatrombopag Maleate Form B (20 mg Avatrombopag equivalent) | 11.3 | 1-20 |
| Lactose Monohydrate | 58.1 | 50-65 |
| Microcrystalline cellulose | 20 | 10-25 |
| Crospovidone Type B | 2.9 | 0.1-10 |
| Colloidal Silicon Dioxide | 2.9 | 0.1-10 |
| Magnesium Stearate | 1 | 0.01-5 |
| **Core tablet weight** | **96.2** | 92-99 |
| **Film Coating:** (Polyvinyl alcohol, Talc, Macrogol, Titanium dioxide, Iron oxide yellow)-Opadry Yellow | 3.8 | 1-8 |
| **TOTAL** | **100** | **100** |

A process for Example 1;
a) Mixing avatrombopag maleate form B, lactose monohydrate, microcrystalline cellulose, crospovidone type b, colloidal silicon dioxide,
b) Sieving the mixture in step (a) through a 630 mm sieve,
c) Adding magnesium stearate to the mixture in step (b),
d) Compressing the homogeneous mixture obtained in such a way that each tablet is of the specified specifications,
e) Coating the core tablet with film coating.

## Claims

1. A film coated tablet formulation comprising avatrombopag maleate or a pharmaceutically acceptable salt, amorphous or crystalline form thereof and at least one filler wherein avatrombopag maleate is crystalline form B.

2. The film coated tablet formulation according to claim 1, wherein the amount of avatrombopag maleate is between 1.0% and 20.0% by weight of the total formulation.

3. The film coated tablet formulation according to claim 1, wherein fillers are selected from the group comprising microcrystalline cellulose, lactose monohydrate, ammonium alginate, calcium carbonate, calcium phosphate, calcium phosphate dehydrate, neutral pellets, calcium sulfate, cellulose, cellulose acetate, compressible sugar, dextrates, dextrin, dextrose, ethylcellulose, fructose, glyceryl palmitostearate, lactitol, mannitol, polymethacrylates, simethicone, sodium alginate, sodium chloride, sorbitol, sucrose, sugar spheres, sulfobutylether beta-cyclodextrin, tragacanth, trehalose, xylitol or mixtures thereof.

4. The film coated tablet formulation according to claim 1 or 3, wherein the filler is microcrystalline cellulose or lactose monohydrate or mixture thereof.

5. The film coated tablet formulation according to claim 3 or 4, wherein the amount of filler is between 70.0% and 90.0% by weight of the total formulation.

6. The film coated tablet formulation according to claim 1, wherein the film coated tablet formulation further comprises at least one pharmaceutically acceptable excipient which is selected from the group comprising disintegrants, lubricants/glidants, film coating agents or a mixture thereof.

7. The film coated tablet formulation according to claim 6, wherein disintegrants are selected from the group comprising crospovidone, croscarmellose sodium, sodium starch glycolate, low-substituted hydroxypropyl cellulose, sodium carboxymethyl cellulose, calcium carboxymethyl cellulose, carboxymethyl cellulose, docusate sodium, guar gum, polyacrylate potassium, sodium alginate, corn starch, alginic acid, alginates, ion-exchange resins, magnesium aluminum silica, sodium dodecyl sulphate, poloxamer, sodium glycine carbonate, sodium lauryl sulphate or mixtures thereof.

8. The film coated tablet formulation according to claim 6 or 7, wherein the disintegrant is crospovidone.

9. The film coated tablet formulation according to claim 7 or 8, wherein the amount of disintegrant is between 0.1% and 10.0% by weight of the total formulation.

10. The film coated tablet formulation according to claim 6, wherein lubricants/glidants are selected from the group comprising from magnesium stearate, colloidal silicon dioxide, talc, calcium stearate, zinc stearate, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, sodium stearyl fumarate, sodium lauryl sulphate or mixtures thereof.

11. The film coated tablet formulation according to claim 6 or 10, wherein the lubricant/glidant is magnesium stearate or colloidal silicon dioxide or mixture thereof.

12. The film coated tablet formulation according to claim 10 or 11, wherein the amount of lubricant/glidant is between 0.01% and 10.0% by weight of the total formulation.

13. A process for preparing a film coated tablet formulation comprising avatrombopag according to any of the preceding claims comprises the following steps;
a) Mixing avatrombopag maleate form B, fillers, disintegrant and lubricant/glidant,
b) Sieving the mixture in step (a),
c) Adding lubricant/glidant to the mixture in step (b),
d) Compressing the homogeneous mixture obtained in such a way that each tablet is of the specified specifications,
e) Coating the core tablet with film coating.
